Europäisches Patentamt

**European Patent Office**

Office européen des brevets

(19)

(11) Publication number: **0 232 556**
**B1**

(12)

# EUROPEAN PATENT SPECIFICATION

(45) Date of publication of the patent specification:
**12.12.90**

(51) Int. Cl.⁵: **C12P 7/56**
// (C12P7/56, C12R1:225)

(21) Application number: **86201876.9**

(22) Date of filing: **27.10.86**

(54) Procedure for the preparation of D-(-)-lactic acid.

(30) Priority: **18.11.85 NL 8503172**

(43) Date of publication of application:
**19.08.87 Bulletin 87/34**

(45) Publication of the grant of the patent:
**12.12.90 Bulletin 90/50**

(84) Designated Contracting States:
**AT BE CH DE ES FR GB IT LI LU NL SE**

(56) References cited:
**EP-A- 0 069 291**
**EP-A- 0 072 010**
**GB-A- 1 157 213**

**CHEMICAL ABSTRACTS, vol. 87, no. 17, 24th Oktober 1977, page 515, abstract no. 132339y, Columbus, Ohio, US; & PL-A-86 492 (AKADEMIA ROLNICZO-TECHNICZNA) 15-07-1976**
**FERMENTED FRESH MILK PRODUCTS, VOL. 1, YOGHURT, J.LJ.RASIC AND J.A.KURMANN, 1978, TECHNICAL DAIRY PUBLISHING HOUSE, DENMARK, PAGES 28-33 AND 46-55**
**BERGEY'S MANUAL OF DETERMINATIVE BACTERIOLOGY, 8TH EDITION (1974), TWO TITLE PAGES AND PAGES 580,582.**
**NIZO-MEDEDELINGEN NR. 7 (FEBRUARY 1973), TITLE**

(73) Proprietor: **COÖPERATIEVE WEIPRODUKTENFABRIEK "BORCULO" W.A., Needseweg 23, NL-7271 AB Borculo(NL)**

(84) Designated Contracting States: **BE CH DE ES FR GB IT LI LU SE AT**

(73) Proprietor: **STICHTING NEDERLANDS INSTITUUT VOOR ZUIVELONDERZOEK, Kernhemseweg 2 P.O.Box 20, NL-6710 BA Ede(NL)**

(84) Designated Contracting States: **NL**

(72) Inventor: **Veringa, Hubertus Antonius, Wuustersgoed 8, NL-6721 XW Bennekom(NL)**

(74) Representative: **van der Beek, George Frans, Ir. et al, Nederlandsch Octrooibureau Scheveningseweg 82 P.O. Box 29720, NL-2502 LS 's-Gravenhage(NL)**

(56) References cited: (continuation)
**PAGE, TABLE OF CONTENTS AND PAGE 90**
**Dellaglio et al., J.Gen.Microbiol, 74, 289-297 (1973).**
**Turner and Martley,**
**Appl.Environm.Microbiol. 45, 1932-1934 (1983).**
**Bergey's Manual of Systematic Bacteriology Volume 2 (1986), two title pages and pages XV, 1220 and 1222**

ACTORUM AG

**Description**

The invention relates to a procedure for the preparation of D-(-)-lactic acid by cultivating a strain of Lactobacillusbulgaricus in a medium which contains lactose or a hydrolysis product thereof.

Such a procedure is known from the European Patent Application No. 0,072,010. In said known procedure use is made of the L. bulgaricus strain DSM 2129 which appears to be capable of producing D-(-)-lactic acid in high yield in a relatively short time from lactose or glucose. It appears from the description, however, that the strain is not capable of producing acid from galactose, which means that only the glucose part of the lactose is used for the production of the lactic acid and the galactose part remains unchanged.

Hitherto, no strains of Lactobacillus species were known which are galactose-positive and produce D-(-)-lactic acid almost exclusively. It is true that it is reported in Bergey's Manual of Determinative Bacteriology (8th edition) that L. bulgaricus is galactose-positive and produces D-(-)-lactic acid almost exclusively, but later investigation revealed that the type strain ATCC 11842 cannot convert any galactose into acid [J. Gen. Microbiol. 74, 289-297 (1973)]. This is confirmed also in the Bergey's Manual of Systematic Bacteriology Vol. 2, 1986, P.H.A. Sneath et al. eds, Williams & Wilkins, Baltimore USA, pp. 1220–1222. Furthermore, it appears from Appl. Environm. Microbiol. 45, 1932-1934 (1983) that strains of L. helveticus and L. bulgaricus which are galactose-positive always produce a mixture of D- and L-lactic acid in which the L-isomer predominates, and that strains of L. lactis and L. bulgaricus which are galactose-negative always produce D-(-)-lactic acid almost exclusively.

Surprisingly, strains of L. bulgaricus have now been found which are in fact capable of converting lactose and the hydrolysis products thereof (glucose and galactose) into D-(-)-lactic acid almost exclusively.

The invention therefore related to a procedure for the preparation of D-(-)-lactic acid as stated in the introduction to this description, characterized in that a strain of Lactobacillusbulgaricus is used which is capable of converting lactose into D-(-)-lactic acid to an extent of more than 50 %.

Good results have been achieved with Lactobacillusbulgaricus strain CBS 743.84 and strain CBS 687.85 and, therefore, the use of said strains or of mutants or variants thereof is preferred. In addition, the strain CBS 688.85 may be mentioned as a suitable strain.

The abovementioned strains of Lactobacillus bulgaricus have been deposited with the Centraal Bureau voor Schimmelcultures in Baarn, the Netherlands. Strain CBS 743.84 is characterized by the following properties:

sugar fermentation: homofermentative
growth at 15°C: -
growth at 45°C: +
acid formed from: amygdalin -
arabinose -
cellobiose -
galactose +
glucose +
lactose +
maltose -
mannitol -
melibiose -
raffinose -
ribose -
sucrose -
salicin -
sorbitol -
trehalose +
xylose -
configuration of the lactic acid formed: D-(-)-.

Said strain is distinguished from the Lactobacillusbulgaricus strains isolated from yoghurt by the ability to ferment galactose and trehalose. The present strain is distinguished from the known Lactobacillus bulgaricus strains which are capable of fermenting galactose by the property of producing D-(-)-lactic acid almost exclusively.

The fermentation of sugar to more than 50% D-(-)-lactic acid by strain CBS 743.84 is not limited to the conversion of lactose: glucose and galactose are also converted into D-(-)-lactic acid.

Instead of the abovenamed strains, according to the invention any variant or mutant thereof may used provided it is capable of converting lactose into D-(-)- lactic acid to an extent of more than 50%.

The raw material which is used for the preparation of D-(-)lactic acid has to contain at least lactose or galactose but it may also contain other sugars, such as glucose, to be converted into D-(-)-lactic acid by the microorganism. The most important use is in the preparation of D-(-)-lactic acid from lactose. In particular milk whey which is available in large quantities as a byproduct of cheese and casein preparation is used as a source of lactose. However, the starting product may be a raw material derived from milk, for example a solution or suspension of lactose.

Additional lactose may also be added to other lactose containing products derived from milk such as skimmed milk, an ultrafiltration permeate of milk or milk whey, dissolved whey powder, or even whey which does not usually contain more than approximately 45 g of lactose per litre, in order to achieve a high lactic acid content. The addition of extra lactose can take place both before and during the process, all at once or in batches.

The cultivation of the microorganism is carried out in the manner known for L. bulgaricus. In addition to lactose and/or galactose and possibly other fermentable sugars, the medium preferably contains also a source of nitrogen and other substances promoting the growth such as vitamins. Meat extract or, for example, milk protein may serve as the source of nitrogen. The addition of yeast extract as a source of vitamins, trace elements and amino acids is preferred. The growth of the microorganism may also be stimulated by adding formate, carbonate and catalase.

Since the D-(-)-lactic acid produced retards the growth of the microorganism, it is desirable to neutralize the acid during the fermentation in a manner such that the pH is held at a value of at least 5.0, preferably at a value of 6.0-7.0. This can be done in a known manner by gradually adding a base such as ammonia, a hydroxide such as sodium, potassium or calcium hydroxide (lime water) or alkali-metal or alkaline-earth metal carbonates, in particular calcium carbonate. A calculated quantity of calcium carbonate can be added all at once at the beginning of the fermentation. It gradually dissolves as more D-(-)-lactic acid is formed. As a result the pH remains in the required region.

It may also be beneficial to add a surface active agent such as an epoxyethane or epoxypropane addition product of a long chain alcohol or acid. Examples of said agents are the products which are marketed under the trademarks of Tween® and Span®.

The isolation of the lactic acid from the fermentation medium is also carried out in a manner known per se. Thus, if a calcium compound, for example calcium carbonate has been used to control the pH during the fermentation, the calcium lactate produced can be converted into D-(-)-lactic acid via reaction with sulphuric acid, the calcium sulphate formed being filtered off and the filtrate concentrated by evaporation.

The invention is explained in more detail on the basis of the following examples.

Example I

A medium I was prepared on the basis of whey ultrafiltration permeate. To said permeate (containing 5% (m/v) dry substance) the following were added:
10% (v/v) skimmed milk;
1% (m/v) yeast extract;
0.1% (v/v) Tween-80®;
phosphate (1.1 g of $Na_2HPO_4.2H_2O$ + 1.2 g of $KH_2PO_4$ per litre);
0.002% (m/v) sodium formate;
0.01% (m/v) sodium carbonate;
catalase (2 Keil units per litre).
The lactose content of said medium was approximately 4.3% (m/v).

After pasteurizing the medium and cooling it to cultivation temperature (37°C) in a cultivation vessel, the medium was inoculated with 1% (v/v) culture of Lactobacillus bulgaricus, strain CBS 743.84; the pH of the mixture was adjusted to a value of 6.0 by means of an ammonia solution. The pH was kept at the value of 6.0 during the cultivation by automatic metered addition of the ammonia solution, the mixture being continuously stirred. During the cultivation the conversion of the lactose was followed by measuring the consumption of ammonia. From the consumption of ammonia it was possible to calculate that if each molecule of lactose were to produce four molecules of lactic acid, 89.8% lactose had been converted after 22 hours. Enzymatic determination of the lactic acid formed indicated that after 22 hours over 85% of the quantity of lactose present at the start had in fact been converted into lactic acid.

Over 99% of the lactic acid formed appeared to consist of the D-(-)-isomer.

Example II

A medium II was prepared consisting of centrifuged mixed whey to which the following were added:
10% (v/v) skimmed milk;
1% (m/v) yeast extract;
0.1% (v/v) Tween-80®;
phosphate (1.1 g of $Na_2HPO_4.2H_2O$ + 1.2 g of $KH_2PO_4$ per litre);
0.008% (m/v) sodium formate;
0.01 % (m/v) sodium carbonate;
catalase (4 Keil units per litre).
The lactose content of the medium was raised to 9.26% (m/v) by adding lactose.

Lactobacillus bulgaricus, strain CBS 743.84, was cultivated in said medium in the manner as indicated in Example I.

At set times the lactose conversion percentage was calculated from the quantity of ammonia consumed at that instant; in addition, the quantities of lactose, galactose and lactic acid were measured at some time instants. Some of the results are summarized in Table A.

The quantities of lactic acid quoted therein have been corrected for the quantities of lactic acid already present at the beginning of the test. Column A records the lactose conversion percentage calculated from the consumption of ammonia, column B records the calculated quantity of lactic acid which would have been produced from the quantity of lactose converted after correction for the residual quantity of galactose; column C gives the quantity of lactic acid actually formed as a percentage of the corresponding calculated quantity in column B.

TABLE A

| Cultivation time (h) at 37°C | Content (g/l) lactose | Content (g/l) galactose | Lactic acid produced (g/l) D(-) | L(+) | Total | A (%) | B (g/l) | C (%) |
|---|---|---|---|---|---|---|---|---|
| 0 | 92.6 | 0.6 | 0 | 0 | 0 | 0 | 0 | |
| 16 1/4 | 12.4 | 27.6 | 53.5 | 2.2 | 55.7 | 60.5 | 57.4 | 97 |
| 19 1/2 | | | | | | 71.5 | | |
| 21 1/2 | | | | | | 77.3 | | |
| 24 1/4 | 4.1 | 11.6 | 77.0 | 3.8 | 80.8 | 85.0 | 82.2 | 98 |
| 40 1/4 | 0.1 | 2.6 | 80.3 | 5.6 | 85.9 | 100.6 | 95.4 | 90 |
| 48 1/2 | | | | | | 100.6 | | |

Example III

A semi-synthetic medium (TGV) was prepared which consisted of:
1% (m/v) trypton;
0.3% (m/v) meat extract;
0.5% (m/v) yeast extract;
4% (v/v) tomato juice;
0.1% (v/v) Tween-80®;
0.2% (m/v) of $K_2HPO_4$.

In addition, in one case 4.1% (m/v) lactose was added (TGV lactose), and in another case 4.5% (m/v) glucose (TGV glucose).

Lactobacillus bulgaricus, strain CBS 743.84, was cultivated in said media in the manner indicated in Example I, but at 44°C instead of at 37°C.

At set times the lactose conversion percentage was calculated from the quantity of ammonia consumed at that instant. After 26 hours 94% lactose was found to have been converted, and after 42 hours around 98%; glucose had already been completely converted after 26 hours. Some other results of said experiment are quoted in Table B. Column A records the lactose conversion percentage calculated from the consumption of ammonia, and column B the calculated quantity of lactic acid which would have been produced from the quantity of lactose converted, after correction for the residual quantity of galactose . Column C gives the quantity of lactic acid actually formed as a percentage of the calculated quantity in column B.

## TABLE B

| Medium | Culti-vation time(h) at 44°C | Lactose g/l | Galactose g/l | Glucose g/l | Lactic acid produced (g/l) | | | A % | B g/l | C % |
|---|---|---|---|---|---|---|---|---|---|---|
| | | | | | D(-) | L(+) | total | | | |
| TGV lactose | 0 | 40.7 | 0.3 | 0.5 | 0 | 0 | 0 | 0 | | |
| | 27 | 0.1 | 2.0 | 0.0 | 34.7 | 0.1 | 34.8 | 97 | 41.5 | 84 |
| TGV glucose | 0 | 0.6 | 0.3 | 45.3 | 0 | 0 | 0 | 0 | | |
| | 27 | 0.0 | 0.4 | 0.0 | 35.6 | 1.2 | 36.8 | 101 | 45.8 | 80 |

Example IV

Various pasteurized media (a-k) were inoculated with 1% (v/v) of a culture of Lactobacillus bulgaricus, strain CBS 743.84. At set times during the cultivation at 37°C the degree of acidity and the pH were determined (in the tests in this example the pH was therefore not kept constant). The increase in the degree of acidity (expressed in °N: the number of ml of 0.1 N hydroxide necessary to neutralize 100 ml of mixture) was taken as a measure of the lactic acid formation.

The compositions of the media used are given in Table C and the results are reproduced in Table D.

## TABLE C

| Constituents | Media | | | | | | | | | | |
|---|---|---|---|---|---|---|---|---|---|---|---|
| | a | b | c | d | e | f | g | h | i | j | k |
| Lactose, Analar, 10% (m/v) | + | - | - | - | - | - | - | - | - | - | - |
| Lactose, "edible quality", 10% (m/v) | - | + | + | + | + | + | + | + | + | + | + |
| Sodium formate, 0.002% (m/v) | - | - | + | + | + | + | + | + | + | + | + |
| Tween-80®, 0.1% (v/v) | - | - | + | + | + | + | + | + | + | + | + |
| Sodium carbonate, 0.1% (v/v) | - | - | + | + | + | + | + | + | + | + | + |
| 4.4 g Na$_2$HPO$_4$.2H$_2$O + 4.8 g KH$_2$PO$_4$ per litre | - | - | - | + | + | + | + | + | + | + | + |
| Skimmed milk, 10% (v/v) | - | - | - | - | + | - | + | - | + | - | + |
| Catalase (2 Keil units per litre) | - | - | - | - | - | + | + | - | - | + | + |
| Yeast extract, 1% (m/v) | - | - | - | - | - | - | - | + | + | + | + |

### TABLE D

| Medium | pH after cultivation time of | | | |
|---|---|---|---|---|
| | 0 h | 19 h | 23.5 h | 43 h |
| a | 5.96 | 4.40 | 4.38 | 4.30 |
| b | 5.77 | 4.43 | 4.37 | 4.37 |
| c | 7.91 | 7.53 | 7.51 | 7.49 |
| d | 6.77 | 6.76 | 6.73 | 6.69 |
| e | 6.75 | 6.72 | 6.70 | 6.63 |
| f | 6.75 | 6.65 | 6.62 | 6.58 |
| g | 6.73 | 5.85 | 4.92 | 4.11 |
| h | 6.73 | 4.18 | 4.12 | 4.01 |
| i | 6.71 | 4.15 | 4.03 | 3.93 |
| j | 6.72 | 4.14 | 4.06 | 4.01 |
| k | 6.71 | 4.04 | 3.91 | 3.86 |

| Medium | Degree of acidity after cultivation time of | | | |
|---|---|---|---|---|
| | 0 h | 19 h | 23.5 h | 43 h |
| a | 1.6 | 2.3 | 2.6 | 2.4 |
| b | 0.9 | 1.6 | 1.9 | 1.8 |
| c | 0.4 | 0.4 | 0.3 | 0.2 |
| d | 28.9 | 29.0 | 28.7 | 28.2 |
| e | 31.2 | 32.0 | 32.0 | 31.8 |
| f | 28.9 | 30.5 | 31.2 | 31.1 |
| g | 30.8 | 50.3 | 61.3 | 76.0 |
| h | 35.8 | 88.3 | 88.6 | 90.2 |
| i | 37.2 | 99.0 | 105.1 | 110.2 |
| j | 34.7 | 89.2 | 89.1 | 90.1 |
| k | 37.6 | 107.4 | 113.5 | 115.3 |

Example V

A medium was prepared which had the same composition as medium II in Example II, but with the difference that the lactose content of the medium was raised not to 9.26%, but to 7.5% (m/v) by adding lactose.
Lactobacillus bulgaricus CBS 743.84 was cultivated in said medium in the manner described in Example I. The conversion was determined by measuring the consumption of ammonia. After 23 h, when 97% of the lactose was found to have been converted, a further 1% (m/v) lactose was added, 100% of which quantity was found to have been converted after 41 h.

# EP 0 232 556 B1

## Claims

1. Procedure for preparing D-(-)-lactic acid by cultivating a strain of <u>Lactobacillus bulgaricus</u> in a medium which contains lactose or a hydrolysis product thereof, characterized in that a <u>galactose-positive</u> strain of <u>Lactobacillus bulgaricus</u> is used which is capable of converting lactose into D-(-)-lactic acid to an extent of more than 50%.

2. Procedure according to Claim 1, characterized in that <u>Lactobacillus bulgaricus</u> strain CBS 743.84, strain CBS 687.85, strain CBS 688.85 or a mutant or variant thereof retaining the ability of converting lactose into D-(-)lactic acid to an extent of more than 50% is used.

3. Procedure according to Claim 1 or 2, characterized in that the medium comprises a lactose containing product derived from milk.

4. Procedure according to Claim 3, characterized in that the medium comprises milk whey.

5. Procedure according to Claim 3, characterized in that the medium comprises an ultrafiltration permeate of milk whey.

6. Procedure according to Claim 3, 4 or 5, characterized in that, in addition to the product derived from milk, the medium contains an additional quantity of lactose.

7. Procedure according to Claims 1-6, characterized in that lactose is added to the medium during the cultivation.

8. Procedure according to Claims 1-7, characterized in that the medium contains growth-stimulating substances in addition.

9. Procedure according to Claims 1-8, characterized in that the cultivation is carried out at pH of at least 5.0, preferably at a pH of 6.0-7.0.

10. Procedure according to Claims 1-9, characterized in that the cultivation is carried out at a temperature of 30-45°C, preferably of 35-40°C.

## Patentansprüche

1. Verfahren zur Herstellung von D–(–)-Milchsäure durch Kultivierung eines Stammes von <u>Lactobacillus bulgaricus</u> in einem Medium, welches Lactose oder ein Hydrolyseprodukt hiervon enthält, dadurch gekennzeichnet, daß ein galactosepositiver Stamm von <u>Lactobacillus bulgaricus</u> verwendet wird, der in der Lage ist, Lactose in einem Ausmaß von mehr als 50% in D–(–)-Milchsäure umzuwandeln.

2. Verfahren nach Anspruch 1, dadurch gekennzeichnet, daß der <u>Lactobacillus bulgaricus</u> Stamm CBS 743.84, Stamm CBS 687.85, Stamm 688.85 oder eine Mutante oder Variante hiervon, bei denen die Fähigkeit erhalten ist, Lactose in einem Ausmaß von mehr als 50% in D–(–)-Milchsäure umzuwandeln, verwendet wird.

3. Verfahren nach Anspruch 1 oder 2, dadurch gekennzeichnet, daß das Medium ein aus Milch gewonnenes lactosehaltiges Produkte enthält.

4. Verfahren nach Anspruch 3, dadurch gekennzeichnet, daß das Medium Milchmolke enthält.

5. Verfahren nach Anspruch 3, dadurch gekennzeichnet, daß das Medium ein Ultrafiltrationspermeat von Milchmolke enthält.

6. Verfahren nach Anspruch 3, 4 oder 5, dadurch gekennzeichnet, daß das Medium zusätzlich zu dem aus Milch gewonnenen Produkt eine zusätzliche Menge von Lactose enthält.

7. Verfahren nach den Ansprüchen 1 bis 6, dadurch gekennzeichnet, daß das Medium während der Kultivierung Lactose zugesetzt wird.

8. Verfahren nach den Ansprüchen 1 bis 7, dadurch gekennzeichnet, daß das Medium zusätzlich wachstumsstimulierende Substanzen enthält.

9. Verfahren nach den Ansprüchen 1 bis 7, dadurch gekennzeichnet, daß die Kultivierung bei einer Temperatur von 30 bis 45°C durchgeführt wird.

10. Verfahren nach den Ansprüchen 1 bis 9, dadurch gekennzeichnet, daß die Kultivierung bei einer Temperatur von 30 bis 45°C, vorzugsweise 35 bis 40°C durchgeführt wird.

## Revendications

1. Procédé de préparation d'acide D–(–)-lactique par culture d'une souche de <u>Lactobacillus bulgaricus</u> dans un milieu qui contient du lactose ou un de ses produits d'hydrolyse, caractérisé en ce qu'on utilise un souche galactose positive de <u>Lactobacillus bulgaricus</u> qui est capable de transformer le lactose en acide D–(–)-lactique dans une mesure supérieure à 50%.

2. Procédé selon la revendication 1, caractérisé en ce qu'on utilise le <u>Lactobacillus bulgaricus</u> souche CBS 743.84, souche CBS 687.85, souche CBS 688.85, ou un de ses mutants ou variantes conservant l'aptitude à transformer le lactose en acide D–(–)-lactique dans une mesure supérieure à 50%.

3. Procédé selon la revendication 1 ou 2, caractérisé en ce que le milieu comprend un produit comprenant du lactose dérivé du lait.

4. Procédé selon la revendication 3, caractérisé en ce que le milieu contient du petit-lait.

5. Procédé selon la revendication 3, caractérisé en ce que le milieu comprend un perméat d'ultrafiltration de petit-lait.

7

6. Procédé selon la revendication 3, 4 ou 5, caractérisé en ce que, outre le produit dérivé du lait, le milieu contient une quantité supplémentaire de lactose.

7. Procédé selon les revendications 1–6, caractérisé en ce qu'on ajoute du lactose au milieu pendant la culture.

8. Procédé selon les revendications 1–7, caractérisé en ce que le milieu contient en outre des substances stimulant la croissance.

9. Procédé selon les revendications 1–8, caractérisé en ce que la culture est conduite à un pH d'au moins 5,0, de préférence de 6,0–7,0.

10. Procédé selon les revendications 1–9, caractérisé en ce que la culture est conduite à une température de 30–45°C, de préférence de 35 à 40°C.